# EUROPÄISCHE PATENTSCHRIFT

(11) **EP 2 877 153 B1**
(45) Veröffentlichungstag und Bekanntmachung des Hinweises auf die Patenterteilung: **13.09.2017**
(21) Anmeldenummer: 13752596.0
(22) Anmeldetag: 23.07.2013
(51) Int. Cl.: A61K 8/67, A61K 8/86, A61K 8/41, A61Q 19/00, A61K 47/26, A61K 31/7056, A61K 31/714, A61P 17/00

(54) **VITAMINZUBEREITUNG**
VITAMIN PREPARATION
PRÉPARATION À BASE DE VITAMINE

(30) Priorität: 24.07.2012 DE 102012014581
(43) Veröffentlichungstag der Anmeldung: 03.06.2015
(73) Patentinhaber: Azoba Health Care AG, 6331 Hünenberg (CH); Mavena International AG, 6331 Hünenberg (CH)
(72) Erfinder: REICHWAGEN, Sven, 59227 Ahlen (DE)
(74) Vertreter: Thiel, Christian
(86) Internationale Anmeldenummer: PCT/EP2013/065477
(87) Internationale Veröffentlichungsnummer: WO 2014/016277

(56) Entgegenhaltungen:
- WO-A1-2005/094842
- WO-A2-02/062260
- WO-A2-2005/120256
- CN-B- 102 068 453
- Anonymous: "Z.Bigatti Delicate Skin Duo - 20% Off Code FAB20", , 3. Februar 2012 (2012-02-03), XP055101594, Gefunden im Internet: URL:http://www.skinstore.com/p-7633-zbigat ti-delicate-skin-duo.aspx [gefunden am 2014-02-12]
- STÜCKER M ET AL: "Topical vitamin B12--a new therapeutic approach in atopic dermatitis-evaluation of efficacy and tolerability in a randomized placebo-controlled multicentre clinical trial", BRITISH JOURNAL OF DERMATOLOGY, OXFORD : WILEY-BLACKWELL, UK, Bd. 150, Nr. 5, 1. Mai 2004 (2004-05-01), Seiten 977-983, XP007910269, ISSN: 0007-0963, DOI: 10.1111/J.1365-2133.2004.05866.X

## Beschreibung

Die Erfindung betrifft eine Vitaminzubereitung, insbesondere in Gelform zur Applikation auf die Haut, die eine physiologisch wirksame Menge an Vitamin B12 oder eines Vorläufers davon enthält.

Vitamin B12 gehört zur Gruppe der Cobalamine und ist ein essentielles Vitamin, das in menschlichen oder tierischen Körpern nicht synthetisiert werden kann. Der menschliche Bedarf muss daher über die Nahrung gedeckt werden. Cobalamine sind insbesondere in tierischer Nahrung in ausreichender Menge enthalten. Der Ursprung ist aber immer bakterieller Natur.

In der Regel wird unter Vitamin B12 Cyanocobalamin verstanden. Weitere Formen sind z. B. das Aquocobalamin, das Hydroxycobalamin und das Methylcobalamin, das in der Regel in den Körperzellen vorkommt. Die als Coenzym B12 wirksame Form ist das 5'-Desoxiadenosylcobalamin. Im Körper werden die aktiven Formen Methylcobalamin und Adenosylcobalamin aus den anderen Formen gebildet.

Erfindungsgemäß wird entsprechend unter Vitamin B12 insbesondere das herkömmlich als Vitamin B12 bezeichnete Cyanocobalamin verstanden, jedoch auch die anderen Vorläuferformen von Coenzym B12, wie sie oben genannt sind.

Vitamin B12 hat sich bei der Behandlung einer Reihe von Hauterkrankungen als sehr wirksam erwiesen. Es wird in Salbenform bei einer Reihe von Dermatosen, aber auch bei atopischen Ekzemen angewandt. Als Salbengrundlage dient ein synthetisches oder pflanzliches Öl oder Fett, insbesondere Avocadoöl.

Mit den herkömmlichen Salben auf Öl- oder Fettbasis wird zwar eine gute Verteilung des Wirkstoffs auf den damit behandelten Hautteilen erreicht, jedoch bleibt es bei einer rein oberflächlichen Verteilung. Ein nennenswerter transdermaler Effekt wird nicht erzielt.

Aufgabe der Erfindung ist daher die Bereitstellung einer Vitaminzubereitung, die geeignet ist, Vitamin B12 auch in die oberen Hautschichten einzubringen, um dort einen nachhaltigen Effekt zu erzielen.

Dieses Ziel wird mit einer Vitaminzubereitung der eingangs genannten Art erreicht, die eine physiologisch wirksame Menge an Vitamin B12 oder eines anderen Cobalamins, ein Polysorbat sowie übliche Trägermaterialien zur Ausbildung einer Trägermatrix enthält.

Polysorbate sind oberflächenaktive Substanzen, die über polare und unpolare Bestandteile verfügen. Aufgrund ihres Aufbaus sind sie geeignet, als Schleppsubstanzen zu dienen. Von dieser Eigenschaft wird hier Gebrauch gemacht. Als Polysorbat kommen die üblichen im Handelt befindlichen Produkte in Frage. Es handelt sich dabei um mehrfach ethoxilierte Sorbitanfettsäureester verschiedener Fettsäuren, etwa der Laurinsäure, Palmitinsäure, Stearinsäure, Ölsäure und Isostearinsäure. Es kann sich dabei um Monoester oder auch Triester handeln. Die Zahl der Polyoxyethyleneinheiten ist variabel, beträgt aber in der Regel 4, 5 oder 20.

Erfindungsgemäß bevorzugtes Polysorbat ist Polysorbat 20, ein Polyoxyethylensorbitanmonolaurat mit 20 Oxyethyleneinheiten, das auch unter der Bezeichnet Tween^{®} bekannt ist.

Neben dem Vitamin B12 oder einem anderen Vorläufer des Coenzyms B12 enthält die erfindungsgemäße Vitaminzubereitung übliche Trägermaterialien zur Ausbildung der Trägermatrix, insbesondere einer Gelmatrix. Solche Stoffe sind beispielsweise Glycerin, Hydroxyethylcellulose, Harnstoff und demineralisiertes Wasser. Zur Einstellung eines pHs, vorzugsweise von pH 5,4, können Zitronensäure und/oder Natronlauge zugegen sein, die sich als ausgesprochen körperverträglich erwiesen haben. Andere Säuren und Basen können ebenfalls verwandt werden. Die Zubereitung kann aber auch als Lotion, Creme, Salbe, Spray, Tinktur oder auch als Shampoo vorliegen.

Die erfindungsgemäße Vitaminzubereitung enthält Vitamin B12 oder eine andere Form von Cobalamin in einer Menge von 40 bis 100 mg, insbesondere in einer Menge von 70 mg pro 100 g der Zubereitung. Das Polysorbat ist einer Menge von 20 bis 100 mg pro 100 g der Zubereitung enthalten, insbesondere in einer Menge von 40 mg.

Zur Gelbildung wird insbesondere Hydroxyethylcellulose 400 in einer Menge von 1000 mg bis 5000 mg pro 100 g der Zubereitung eingesetzt. Weitere Bestandteile können Glycerin in einer Menge von 1000 mg bis 5000 mg pro 100 g sowie Harnstoff in einer Menge von 2000 mg bis 7000 mg pro 100 g sein.

Neben den oben genannten Bestandteilen kann die Zubereitung des Weiteren ein Antiseptikum enthalten, etwa Polyhexanid. Dieses wird in Form eines kommerziell erhältlichen Lösungskonzentrats (20 % m/V) zugesetzt und ist vorzugsweise in einer Menge von 100 bis 500 mg Konzentrat pro 100 g enthalten. Der Polyhexanidzusatz ist insbesondere bei entzündeten und entzündlichen Hauterkrankungen, bei denen es zu einem Befall mit Mikroorganismen kommt, hilfreich. Polyhexanid ist ein Antiseptikum auf Basis von Polyhexamethylenbiguanidin (PHMB).

Die Vitaminzubereitung kann weitere für die Behandlung der Haut vorteilhafte Wirkstoffe enthalten. Zu nennen ist beispielsweise Dexpanthenol, das ebenfalls ein Vitaminvorläufer ist. Es wird im Körper zu Pantothensäure umgewandelt, die ein Vitamin aus der Gruppe der B-Vitamine (Vitamin B5) ist.

Dexpanthenol ist beispielsweise in einer Menge von 1000 mg bis 10000 mg pro 100 g in der Zubereitung enthalten. Weitere Bestandteile sind ggf. Polyethylenglykol 400 sowie, also Antioxidanz, Natriumascorbat.

Es können weitere, in der Behandlung von Hauterkrankungen bewährte Stoffe in der Formulierung enthalten sein. Zu nennen wären Taurin, Koffein, Lysin, Kreatin, aber auch Zinksalze, etwa Zinksulfat. Insbesondere Zinksulfat ist ein altbekanntes Mittel bei der Behandlung von Ekzemen und kann beispielsweise in einer Menge von 50 bis 1000 mg/100 g enthalten sein.

Die erfindungsgemäße Vitaminzubereitung setzt sich vorzugsweise wie folgt zusammen:
50 bis 500 mg Polyhexanid in 20 %iger wässeriger Lösung
20 bis 100 mg Polysorbat
1000 bis 5000 mg Glycerin
500 bis 5000 mg Hydroxyethylcellulose 400
2500 bis 7000 mg Harnstoff
Zitronensäure zum Puffern auf pH 5,4
NaOH zum Puffern auf pH 5,4 sowie
demineralisiertes Wasser ad 100 g enthält.

Die erfindungsgemäße Vitaminzubereitung kann als Gel auf die Haut aufgetragen werden, aber auch in Gelform auf bzw. in einem Pflaster oder Pad enthalten sein, das dann auf die Haut aufgebracht wird. Sie kann ferner als Spray aus- und aufgebracht werden oder als Tinktur beispielsweise in Form von Nasenspray oder von Augentropfen. Neben der Wirksamkeit bei Dermatosen und Ekzemen wurde eine gute Wirkung bei andersartigen Reizungen der Haut und Schleimhäute festgestellt, insbesondere auch bei allergisch bedingten Reizungen, wie Pollenallergien, Hausstauballergie und allergischen Reaktionen auf Insektenstiche.

Eine weitere Anwendung ergibt sich bei der Reinigung, Pflege und Reizarmen Imprägnierung von Kontaktlinsen.

Die Erfindung wird durch die nachfolgenden Beispiele näher erläutert:

### Beispiel 1:

Eine Vitaminzubereitung in Gelform wurde aus den folgenden Bestandteilen hergestellt:
1. Vitamin B12 (Cyanocobalamin) 70 mg
2. Polyhexanid-Lösungskonzentrat 20 % (m/V) 200 mg
3. Polysorbat 20 40 mg
4. Glycerin 2500 mg
5. Hydroxyethylcellulose 400 bis zu 5000 mg, je nach gewünschter Konsistenz
6. Harnstoff 5000 mg
7. Zitronensäure 300 mg zum Puffern auf pH 5,4
8. ggf. NaOH zum Puffern auf pH 5,4
9. demineralisiertes Wasser ad 100 g

Die Bestandteile werden unter leichter Erwärmung gemischt und zu einem Gel miteinander verrührt.

### Beispiel 2:

Die Zubereitung von Beispiel 1 wird um 5000 mg Dexpanthenol und 2000 mg PEG400 ergänzt.

Als weitere Bestandteile können etwa 300 mg Taurin, 25 mg Koffein, 1000 mg Lysin, 500 g Zinksulfat und/oder 500 mg Kreatin eingemischt werden. Natriumascorbat wird zugemischt, soweit ein Antioxidanz benötigt wird.

### Beispiel 3:

Antiallergische Augentropfen wurden wie folgt zubereitet:
1. Vitamin B12 70 mg
2. Dexpanthenol 500 mg
3. Polyhexanid (Lösungskonzentrat 20 % (m/V)) 0,75 mg
4. Polysorbat 20 40 mg
5. HPMC 10 mg
6. Trinatriumcitrat 10 mg
7. Zitronensäure ad pH 7,2
8. Natriumhydroxid ad pH 7,2
9. Natriumchlorid zur Einstellung auf 300 mosmol
10.demineralisiertes Wasser ad 100 g

### Beispiel 4:

Ein Mittel für die Kontaktlinsenpflege wurde wie folgt hergestellt:
1. Vitamin B12 70 mg
2. Polyhexanid (Lösungskonzentrat 20 % (m/V)) 0,75 mg
4. Polysorbat 20 40 mg
5. HPMC 10 mg
6. Trinatriumcitrat 10 mg
7. Zitronensäure ad pH 7,2
8. Natriumhydroxid ad pH 7,2
9. Natriumchlorid zur Einstellung auf 300 mosmol
10.demineralisiertes Wasser ad 100 g

### Beispiel 5:

Nasenspray/Tropfen wurden nach folgender Rezeptur hergestellt:
1. Vitamin B12 (Cyanocobalamin) 70 mg
2. Dexpanthenol 500 mg
3. Polyhexanid (Lösungskonzentrat 20 % (m/V)) 0,15 mg
4. Polysorbat 20 40 mg
5. HPMC 100 mg
6. Trinatriumcitrat 25 mg
7. Zitronensäure ad pH 7,2
8. Natriumchlorid 0,9 bis 2,0 g für die Einstellung auf eine hypatrone schnell abschwellende Lösung
9. demineralisiertes Wasser ad 100 g

### Patientenstudie:

Sieben an starker Neurodermitis leidenden Patienten mit Ekzemen an Handflächen, Armen und teilweise im Gesicht bei erheblichem Juckreiz wurden mit dem im Beispiel 1 beschriebenen Gel topisch behandelt. Der Juckreiz lässt unmittelbar nach der Behandlung nach und war nach wenigen Minuten verschwunden. Pusteln und Schwellungen verschwanden nach einem Tag, Rötungen spätestens am zweiten Tag.

Die Anwendung des Gels bei fünf Patienten mit Psoriasis führte zum sofortigen Rückgang des Juckreizes und zum Abklingen der Rötungen am zweiten Tag. Bei zwei Patienten mit Dermatosen an der Kopfhaut verschwanden diese innerhalb von einer Woche.

Eine Zubereitung nach Beispiel 3 wurde zehn Allergikern, die aufgrund von Pollenflug an Bindehautentzündungen der Augen litten, in die Augen verabreicht. Der Juckreiz verschwand augenblicklich, die Entzündungserscheinungen waren spätestens nach drei Stunden weitestgehend verschwunden.

## Patentansprüche

1. Verwendung eines Polysorbats in einer Vitaminzubereitung zur Behandlung von Reizzuständen der Haut und Schleimhäute, wobei die Vitaminzubereitung eine physiologisch wirksame Menge an Vitamin B12 oder eines anderen Vorläufers von Adenosylcobalamin (Coenzym B12), das Polysorbat sowie übliche Trägermaterialien zur Ausbildung einer Trägermatrix enthält, als Schleppmittel zur Einbringung in die oberen Hautschichten von Vitamin B12 oder des anderen Vorläufers von Adenosylcobalamin in die Haut.

2. Verwendung nach Anspruch 1, **dadurch gekennzeichnet, dass** das Polysorbat Polysorbat 20 (Tween^{®}) ist.

3. Verwendung nach einem der vorstehenden Ansprüche, **dadurch gekennzeichnet, dass** die Vitaminzubereitung 40 bis 100 mg Vitamin B12 oder eines Vorläufers von (Coenzym B12) auf 100 g der Zubereitung enthält.

4. Verwendung nach einem der vorstehenden Ansprüche, **dadurch gekennzeichnet, dass** die Vitaminzubereitung 20 bis 100 mg Polysorbat auf 100 g der Zubereitung enthält.

5. Verwendung nach einem der vorstehenden Ansprüche, **dadurch gekennzeichnet, dass** die Vitaminzubereitung Polyhexanid enthält.

6. Verwendung nach Anspruch 5, **dadurch gekennzeichnet, dass** die Vitaminzubereitung 50 bis 500 mg einer 20 %igen Polyhexanidlösung auf 100 g der Zubereitung enthält.

7. Verwendung nach einem der vorstehenden Ansprüche, **dadurch gekennzeichnet, dass** die Vitaminzubereitung Harnstoff enthält.

8. Verwendung nach einem der vorstehenden Ansprüche, **dadurch gekennzeichnet, dass** die Vitaminzubereitung ein Zinksalz enthält.

9. Verwendung nach Anspruch 8, **dadurch gekennzeichnet, dass** die Vitaminzubereitung 50 bis 1000 mg Zinksalz pro 100 g der Zubereitung enthält.

10. Verwendung nach einem der vorstehenden Ansprüche, **dadurch gekennzeichnet, dass** die Vitaminzubereitung Taurin, Lysin, Koffein und/oder Kreatin enthält.

11. Verwendung nach einem der vorstehenden Ansprüche, **dadurch gekennzeichnet, dass** die Vitaminzubereitung Dexpanthenol enthält.

12. Verendung nach Anspruch 11, **dadurch gekennzeichnet, dass** die Vitaminzubereitung 500 bis 10000 mg Dexpanthenol auf 100 g der Zubereitung enthält.

13. Verwendung nach einem der Ansprüche 1 bis 12, **dadurch gekennzeichnet, dass** die Vitaminzubereitung in Gelform vorliegt und
40 bis 100 mg Vitamin B 12 oder eines anderen Vorläufers von Coenzym B12
50 bis 500 mg Polyhexanid in 20 %iger wässeriger Lösung
20 bis 100 mg Polysorbat
1000 bis 5000 mg Glycerin
500 bis 5000 mg Hydroxyethylcellulose 400
2500 bis 7000 mg Harnstoff
Zitronensäure zum Puffern auf pH 5,4
NaOH zum Puffern auf pH 5,4 sowie
demineralisiertes Wasser ad 100 g enthält.

14. Verwendung nach Anspruch 13, **dadurch gekennzeichnet, dass** die Zubereitung weiterhin 1000 mg bis 4000 mg PEG400 enthält.

15. Verwendung nach einem der vorstehenden Ansprüche, wobei die Vitaminzubereitung auf/in einem Pad zur Applikation auf die Haut, als Spray oder als Tinktur zur Applikation auf Schleimhäute vorliegt.

## Claims

1. Use of a polysorbate in a vitamin preparation for the treatment of irritations of the skin and mucosas, the vitamin preparation containing a physiologically effective amount of vitamin B12 or of a different precursor of adenosylcobalamin (coenzyme B12), the polysorbate and also customary support materials for the formation of a support matrix, as entraining agent for the introduction into the upper dermal layers of vitamin B12 or of the different precursor of adenosylcobalamin into skin.

2. Use according to Claim 1, **characterized in that** the polysorbate is polysorbate 20 (Tween^{®}).

3. Use according to either of the preceding claims, **characterized in that** the vitamin preparation contains from 40 to 100 mg of vitamin B12 or of a precursor of to 100 g of the preparation.

4. Use according to any of the preceding claims, **characterized in that** the vitamin preparation contains from 20 to 100 mg of polysorbate to 100 g of the preparation.

5. Use according to any of the preceding claims, **characterized in that** the vitamin preparation contains polyhexanide.

6. Use according to Claim 5, **characterized in that** the vitamin preparation contains from 50 to 500 mg of a 20% polyhexanide solution to 100 g of the preparation.

7. Use according to any of the preceding claims, **characterized in that** the vitamin preparation contains urea.

8. Use according to any of the preceding claims, **characterized in that** the vitamin preparation contains a zinc salt.

9. Use according to Claim 8, **characterized in that** the vitamin preparation contains from 50 to 1000 mg of zinc salt per 100 g of the preparation.

10. Use according to any of the preceding claims, **characterized in that** the vitamin preparation contains taurine, lysine, caffeine and/or creatine.

11. Use according to any of the preceding claims, **characterized in that** the vitamin preparation contains dexpanthenol.

12. Use according to Claim 11, **characterized in that** the vitamin preparation contains from 500 to 10 000 mg of dexpanthenol to 100 g of the preparation.

13. Use according to any of Claims 1 to 12, **characterized in that** the vitamin preparation is in gel form and
contains from 40 to 100 mg of vitamin B12 or of a different precursor of coenzyme B12 from 50 to 500 mg of polyhexanide in 20% aqueous solution
from 20 to 100 mg of polysorbate
from 1000 to 5000 mg of glycerol
from 500 to 5000 mg of hydroxyethylcellulose 400
from 2500 to 7000 mg of urea
citric acid for buffering to pH 5.4
NaOH for buffering to pH 5.4 and also
demineralized water to 100 g.

14. Use according to Claim 13, **characterized in that** the preparation further contains from 1000 mg to 4000 mg of PEG400.

15. Use according to any of the preceding claims, wherein the vitamin preparation is present on/in a pad for application to skin, as spray or as tincture for application to mucosas.

## Revendications

1. Utilisation d'un polysorbat dans une préparation de vitamine pour le traitement d'états d'irritation de la peau et des muqueuses, la préparation de vitamine contenant une quantité physiologiquement efficace de vitamine B12 ou d'un autre précurseur d'adénosylcobalamine (coenzyme B12), le polysorbat et des matériaux vecteurs usuels pour la formation d'une matrice vecteur, en tant qu'agent d'entraînement pour l'introduction dans les couches supérieures de la peau de vitamine B12 ou de l'autre précurseur d'adénosylcobalamine dans la peau.

2. Utilisation selon la revendication 1, **caractérisée en ce que** le polysorbat est le polysorbat 20 (Tween®).

3. Utilisation selon l'une quelconque des revendications précédentes, **caractérisée en ce que** la préparation de vitamine contient 40 à 100 mg de vitamine B12 ou d'un précurseur de pour 100 g de la préparation.

4. Utilisation selon l'une quelconque des revendications précédentes, **caractérisée en ce que** la préparation de vitamine contient 20 à 100 mg de polysorbat pour 100 g de la préparation.

5. Utilisation selon l'une quelconque des revendications précédentes, **caractérisée en ce que** la préparation de vitamine contient du polyhexanide.

6. Utilisation selon la revendication 5, **caractérisée en ce que** la préparation de vitamine contient 50 à 500 mg d'une solution de polyhexanide à 20 % pour 100 g de la préparation.

7. Utilisation selon l'une quelconque des revendications précédentes, **caractérisée en ce que** la préparation de vitamine contient de l'urée.

8. Utilisation selon l'une quelconque des revendications précédentes, **caractérisée en ce que** la préparation de vitamine contient un sel de zinc.

9. Utilisation selon la revendication 8, **caractérisée en ce que** la préparation de vitamine contient 50 à 1 000 mg de sel de zinc pour 100 g de la préparation.

10. Utilisation selon l'une quelconque des revendications précédentes, **caractérisée en ce que** la préparation de vitamine contient de la taurine, de la lysine, de la caféine et/ou de la créatine.

11. Utilisation selon l'une quelconque des revendications précédentes, **caractérisée en ce que** la préparation de vitamine contient du dexpanthénol.

12. Utilisation selon la revendication 11, **caractérisée en ce que** la préparation de vitamine contient 500 à 10 000 mg de dexpanthénol pour 100 g de la préparation.

13. Utilisation selon l'une quelconque des revendications 1 à 12, **caractérisée en ce que** la préparation de vitamine se présente sous la forme d'un gel et contient
40 à 100 mg de vitamine B12 ou d'un autre précurseur de coenzyme B12,
50 à 500 mg de polyhexanide dans une solution aqueuse à 20 %,
20 à 100 mg de polysorbat,
1 000 à 5 000 mg de glycérine,
500 à 5 000 mg d'hydroxyéthylcellulose 400,
2 500 à 7 000 mg d'urée,
de l'acide citrique pour le tamponnage à pH 5,4,
du NaOH pour le tamponnage à pH 5,4 et
de l'eau déminéralisée jusqu'à 100 g.

14. Utilisation selon la revendication 13, **caractérisée en ce que** la préparation contient en outre 1 000 mg à 4 000 mg de PEG 400.

15. Utilisation selon l'une quelconque des revendications précédentes, dans laquelle la préparation de vitamine se présente sur/dans un tampon pour l'application sur la peau, sous la forme d'une pulvérisation ou sous la forme d'une teinture pour l'application sur les muqueuses.
